# EUROPEAN PATENT SPECIFICATION

(11) **EP 4 444 815 B1**
(45) Date of publication and mention of the grant of the patent: **29.10.2025**
(21) Application number: 22830243.6
(22) Date of filing: 05.12.2022
(51) Int. Cl.: C09J 153/02, A61F 13/00

(54) **HOT MELT ADHESIVE COMPOSITION WITH STYRENE BLOCK COPOLYMER**
HEISSSCHMELZKLEBSTOFFZUSAMMENSETZUNG MIT STYROLBLOCKCOPOLYMER
COMPOSITION ADHÉSIVE THERMOFUSIBLE COMPORTANT UN COPOLYMÈRE SÉQUENCÉ DE STYRÈNE

(30) Priority: 07.12.2021 EP 21306720
(43) Date of publication of application: 16.10.2024
(73) Proprietor: Bostik SA, 92800 Puteaux (FR)
(72) Inventor: BELLINI, Clement, 60280 VENETTE (FR); DUPLAGA, Urszula, 60280 VENETTE (FR)
(74) Representative: Arkema Patent
(86) International application number: PCT/EP2022/084442
(87) International publication number: WO 2023/104726

(56) References cited:
- EP-A1- 3 760 684
- US-A1- 2013 202 787
- US-A1- 2018 016 478
- US-A1- 2020 377 716

## Description

### FIELD OF THE INVENTION

The present invention relates to a hot melt adhesive composition comprising a styrene block copolymer and a wax.

The present invention also concerns the use of said hot melt adhesive composition as core stabilization adhesive.

### BACKGROUND OF THE INVENTION

Absorbent articles, such as diapers, adult incontinence products, sanitary napkins, etc.., are well known articles of staple manufacturing. Multiple attempts have been made to provide them with an overall good fit and with a high absorbent capacity.

Typical absorbent articles comprises an absorbent core interposed between a topsheet and a backsheet. The absorbent core generally includes absorbent polymer materials which allow to store large amounts of bodily fluids such as for example urine in case of diaper. The absorbent core also comprises adhesives for allowing immobilization of the core structure. These adhesives are different from the adhesives used to bond the absorbent core to the topsheet and to the backsheet of the absorbent article.

Absorbent articles are generally disposable products that can be worn for many hours, in a dry state or even in wet state. To provide good wearing comfort, it is important to keep the absorbent materials/network of the absorbent core comprised by a diaper or other absorbent article in place, both when the article is dry or during expansion while the core is fully/partially wet (urine or other bodily liquids).

Hot melt adhesive compositions are often used to bond substrates together or to contribute to the integrity and functionality of disposable absorbent articles. Some attempts have been made to obtain high cohesion of the absorbent core by using styrene block copolymers with high contents of styrene in hot melt adhesives. However, the viscosities of such hot melt adhesives are too high and makes them difficult to apply on substrates (such as poor processability), and implies to apply them at high temperature which can damage the substrates (such as thin substrates) but also require lots of energy. For example US2013202787A1 describes low application temperature hot melt adhesives which are suitable for bonding an elastomeric material in manufacture of disposable absorbent articles, such as disposable diapers. Such low application temperature hot melt adhesive comprises (a) at least one styrenic block copolymer which has a styrene content greater than 40%, and (b) a wax, wherein the adhesive has a viscosity less than about 8,500 mPa.s at 120° C.

There is thus a need to provide hot melt adhesive compositions having appropriate viscosities (such as good processability) while exhibiting good adhesive and cohesion properties, especially in the absorbent core.

### DESCRIPTION OF THE INVENTION

### Hot melt adhesive composition

The present invention concerns a hot melt adhesive composition comprising:
a) from 22% to 60% by weight of at least one styrene block copolymer (A) (designated as "SBC (A)") comprising:
   - at least one linear styrene block copolymer (A1); and
   - optionally at least one styrene diblock copolymer (A2);
   said styrene block copolymer (A) having:
   - a styrene content higher than or equal to 38% by weight based on the total weight of SBC (A); and
   - a diblock content ranging from 0% to 10% by weight based on the total weight of SBC(A);
b) from 0.1% to 5% by weight of at least one wax having a congealing point higher than or equal to 60°C and a needle penetration at 25°C lower than or equal to 2 dmm;
wherein the hot melt adhesive composition has a viscosity at 120°C higher than or equal to 30 000 mPa.s, and a viscosity at 177°C lower than or equal to 5 000 mPa.s.

### Styrene block copolymer (A)

The styrene block copolymer (A) (designated as "SBC (A)") comprises, preferably consists essentially of, and more preferably consists of:
- at least one linear styrene block copolymer (A1); and
- optionally at least one styrene diblock copolymer (A2);
said styrene block copolymer (A) having:
- a styrene content higher than or equal to 38% by weight based on the total weight of SBC (A); and
- a diblock content ranging from 0% to 10% by weight based on the total weight of SBC(A).

The styrene block copolymer (A) can comprise only linear styrene block copolymer(s) (A1) and no styrene diblock copolymer (A2), or it can comprises a blend of at least one linear styrene block copolymer (A1) with at least one styrene diblock copolymer (A2). Blends may be for example two different linear styrene block copolymers (A1) and one styrene diblock copolymer (A2), or three different linear styrene block copolymers (A1) and two different diblock copolymers (A2), or one linear styrene block copolymer (A1) and one styrene diblock copolymer (A2).

The styrene block copolymer(s) included in SBC (A), in particular (A1) and optional (A2), comprise(s) at least one non elastomeric block A' being a styrene based polymer, and at least one elastomeric block X being a conjugated diene based block polymer, said conjugated diene based block polymer being preferably non-hydrogenated.

The linear styrene block copolymer (A1) ("SBC (A1)") refers to a linear styrene-conjugated diene based block copolymer of at least three blocks.

In particular, the linear SBC (A1) can be a triblock SBC of formula (1) or a multiblock SBC of formula (2) below:

A'-X-A' (1)

A'-(X-A')m-X (2)

wherein:
- A' is a non elastomeric block of styrene based polymer, preferably of polystyrene,
- X is an elastomeric block of conjugated diene block polymer, said conjugated diene being preferably chosen from butadiene, isoprene and mixture thereof,
- m is an integer of at least 1.

The styrene diblock copolymer (A2) ("diblock (A2)") is a styrene-conjugated diene based diblock copolymer, namely a two-block copolymer comprising one non elastomeric block A' being a styrene based polymer (preferably polystyrene) and one elastomeric block X being a conjugated diene based block polymer, said conjugated diene based block polymer being preferably non-hydrogenated.

The styrene diblock copolymer (A2) can be represented by formula (3) below:

A'-X (3)

wherein:
- A' is a non elastomeric block of styrene based polymer, preferably of polystyrene,
- X is an elastomeric block of conjugated diene block polymer, said conjugated diene being preferably chosen from butadiene, isoprene and mixture thereof.

The diblock content in SBC (A) refers to the proportion by weight of styrene diblock copolymer(s) (A2) included in SBC (A).

Preferably, the diblock content ranges from 0% to 5 % by weight, more preferably from 0% to 2% by weight, and even more preferably from 0% to 1% by weight based on the total weight of SBC (A).

The styrene content in SBC (A) refers to the proportion by weight of styrene based polymer blocks included in the styrene block copolymers of SBC (A).

Preferably, the SBC (A) has a styrene content ranging from 38% to 70% by weight, more preferably from 38% to 60% by weight, and even more preferably from 40% to 50% by weight based on the total weight of SBC (A).

In an embodiment, the hot melt adhesive composition comprises a mixture of two or more different styrene block copolymers (A).

In the embodiment wherein the hot melt adhesive composition comprises a mixture of different styrene block copolymers (A) (two or more), the overall styrene content of said mixture may represent at least 40% by weight, preferably from 40% to 60% by weight, and even more preferably from 42% to 50% by weight based on the total weight of said mixture.

In the embodiment wherein the hot melt adhesive composition comprises a mixture of different styrene block copolymers (A) (two or more), the overall diblock content of said mixture may range from 0% to 5% by weight, more preferably from 0% to 2% by weight, and even more preferably from 0% to 1% by weight, based on the total weight of said mixture.

The linear styrene block copolymer (A1) may be chosen from the following linear triblock copolymers: styrene-butadiene-styrene copolymer (SBS), styrene-isoprene-styrene copolymer (SIS), styrene-ethylene-butylene-styrene copolymer (SEBS), styrene-butadiene-butylene-styrene copolymer (SBBS), styrene-ethylene-propylene-styrene copolymer (SEPS), and any mixture thereof. Preferably, the linear styrene block copolymer (A1) is select from the group consisting of styrene-butadiene-styrene copolymer (SBS), styrene-isoprene-styrene copolymer (SIS), and mixtures thereof.

The diblock styrene copolymer (A2) may be chosen from the following diblock copolymers: styrene-butadiene diblock copolymer (SB), styrene-isoprene diblock copolymer (SI), styrene-ethylene-butylene diblock copolymer (SEB), styrene-ethylene-propylene diblock copolymer (SEP), styrene-butylene-butene (SBB), and mixtures thereof.

The conjugated diene based block polymers forming the elastomeric blocks of the linear SBC (A1) and of the optional diblock (A2) (corresponding to X in formulas (1), (2) and (3)) are preferably the same. More preferably, SBC (A) is styrene-butadiene based, meaning that the conjugated diene based block polymers forming the elastomeric blocks of the linear SBC (A1) and of the diblock copolymer (A2) are polybutadiene, and thus that in formulas (3), (4) and (5), B' is an elastomeric block of polybutadiene.

The styrene block copolymer (A) has preferably a melt flow index (200°C/5kg) higher than or equal to 20 g/10 min, more preferably higher than or equal to 30 g/10 min.

In the embodiment wherein the hot melt adhesive comprises a mixture of different block copolymer (A), the average melt flow index of the mixture is preferably higher than or equal to 20 g/10 min (200°C/5kg), even more preferably higher than or equal to 30 g/10 min. The average melt flow index of the mixture is calculated in relation to the weight percentage of each different styrene block copolymer (A) compared to the total weight of the mixture of the styrene block copolymers (A).

The melt flow index may be measured at 200°C/5 kg according to ASTM D1238.

The useful styrene block copolymer (A) can be prepared from at least one substituted or non-substituted styrene monomer and at least one conjugated diene monomer, by any block polymerization method well known in the art to form a mixture of linear type styrene block copolymer and optional styrene diblock copolymer, as used according to the invention.

The useful SBC (A) are also commercially available. By way of example of useful SBC (A) which are commercially available, mention may be made in particular of:
- Vector^{®}4411 sold by TSRC, which is a linear styrene-isoprene-styrene block copolymer having a styrene content ranging of 44% by weight and a diblock content less than 1% by weight;
- Kraton^{®} D1162 sold by KRATON, which is a linear styrene-isoprene-styrene block copolymer having a styrene content ranging of 43% by weight and a diblock content less than 1% by weight;
- Taipol^{®}4202 sold by TSRC, which is a linear styrene-butadiene-styrene block copolymer having a styrene content ranging of 40% by weight and a diblock content less than 1% by weight.

Preferably, the total content of styrene block copolymer(s) (A) ranges from 25% to 50% by weight, more preferably from 25% to 40% by weight, and even more preferably from 27% to 37% by weight based on the total weight of the hot melt adhesive composition.

### Wax

The hot melt adhesive composition comprises from 0.1% to 5 % by weight of at least one wax as defined above, preferably from 1% to 5% by weight, more preferably from 1.5% to 4.0%, and even more preferably from 2.0% to 4.0% by weight based on the total weight of the hot melt adhesive composition.

The wax is preferably a synthetic wax and more preferably a synthetic wax selected from the group consisting of Fischer-Tropsch waxes, polyolefin waxes, and mixtures thereof. Preferably, the wax is a Fischer-Tropsch wax.

As used herein, the term "synthetic polyolefin wax" refers to those polymeric or long-chain entities comprised of olefinic monomer units derived from polymerization.

By *"Fischer-Tropsch wax"* is meant a wax obtained by the so-called Fischer-Tropsch process. The Fischer-Tropsch process includes converting a synthesis gas comprising mainly hydrogen and carbon monoxide to hydrocarbons. The conversion is typically effected by contacting the synthesis gas with a Fischer-Tropsch catalyst, such as iron or cobalt based catalyst, in a fixed bed or a slurry bed reactor under either low or high temperature Fischer-Tropsch operating conditions. In this manner, a mixture of hydrocarbons having different boiling ranges is obtained. The Fischer-Tropsch wax is then recovered, e.g. by means of distillation, from this hydrocarbon mixture.

The wax according to the invention has a congealing point higher than or equal to 60°C, preferably higher than or equal to 65°C, even more preferably higher than or equal to 70°C, and advantageously higher than or equal to 75°C. The wax according to the invention has preferably a congealing point higher than or equal to 80°C.

The congealing point of the wax may be lower than 150°C, preferably lower than 120°C.

The congealing point of the determined according to ASTM D 938.

As used herein, the term "dmm" means decimillimeters.

The needle penetration of the wax is lower than or equal to 2 dmm, preferably lower than or equal to 1 dmm.

As used herein, the terms "needle penetration", "needle penetration hardness", "penetration hardness" are similar.

The needle penetration of the wax is determined according to ASTM D 1321.

The wax may have a melting point of 140°C or less, more preferably ranging from 100 to 120°C.

The melting point of the wax may be determined by ASTM method D127-60.

The wax according to the invention may be a commercially available wax. Examples of commercially available waxes suitable for the present invention include Fischer-Tropsch waxes from Shell sold under the trade names SX105 which has a congealing point of 101-108°C and a needle penetration of 2 dmm, from Sasol sold under the trade name SASOLWAX^{®} H1 which has a congealing point of 97°C and a needle penetration hardness of 1 dmm, or PE wax from SCG Chemicals under the trade name LP1060P which has a congealing point of 116°C and a needle penetration hardness of 2 dmm.

### Plasticizer

The hot melt adhesive composition of the present invention may comprise a plasticizer. The plasticizer may be a solid or a liquid plasticizer.

The hot melt adhesive composition may comprise from about 0% to about 40 %, preferably from about 5% to about 35 %, more preferably from about 10% to about 30 %, by weight of plasticizer(s) relative to the total weight of said hot melt adhesive composition.

The plasticizer may be chosen from the group consisting of naphthenic and paraffinic oils, olefin oligomers, low molecular weight polymers, glycol benzoates, vegetable and animal oils, and derivatives thereof.

Naphthenic oils and paraffinic oils are petroleum-based oils which consist in a mixture of naphthenic hydrocarbons (*e.g*. aliphatic, saturated or unsaturated, C4 to C7-member hydrocarbon rings; preferably aliphatic, saturated or unsaturated, C4 to C6-member rings, including cycloalkanes such as cyclopentane, cyclohexane, cycloheptane), paraffinic hydrocarbons (saturated, linear or branched, alkanes) and aromatic hydrocarbons (aromatic hydrocarbon rings, which may be monocyclic or polycyclic, and preferably aromatic C6-member hydrocarbon rings).

The classification of naphthenic and paraffinic oil is made based on the amount of each type of hydrocarbons in the oil. Typically, paraffinic oils have a paraffinic hydrocarbons content of at least about 50% by weight; naphthenic oils have a naphthenic hydrocarbons content from about 30% to about 50% by weight, relative to the total weight of the plasticizer.

The oligomers may be selected from the group consisting of polypropylene, polybutene, hydrogenated polyisopropene, hydrogenated butadiene, or the like having average molecular weights between about 100 and about 10,000 g/mol.

The vegetable and animal oils may be selected from glycerol esters of usual fatty acids and polymerization products thereof.

In a preferred embodiment, the plasticizer is a naphtenic oil.

Suitable plasticizers are commercially available under the tradenames Nyflex^{®} 223 and Nyflex^{®} 222B (naphtenic oils) from Nynas, Parol^{®} (paraffinic oils), Kaydol^{®} (paraffinic oil) from Sonneborn, Olympus^{®} L500 from Ergon, Primol^{®} 352 and 382 from Exxon Mobil, Calsol^{®} 5550 from Calumet and Karamay N4010 (naphtenic oils) from Petrochina Karamay Petrochemical Co. LTD.

### Tackifying resin

The hot melt adhesive composition may comprise at least one tackifying resin (tackifier).

The hot melt adhesive composition may comprise from 20% to 75 %, preferably from 30% to 70 %, more preferably from 40% to 60 % by weight of tackifying resin(s) relative to the total weight of the composition.

Suitable tackifying resins are those which are compatible with the styrene block copolymer (A).

The tackifying resin may be selected from the group consisting of:
- i) the homopolymers of terpene resins preferably having a softening point from 10°C to 120°C (for example obtained by polymerization of terpene hydrocarbons such as mono-terpene (or pinene) optionally in presence of Friedel-Crafts catalysts;
- ii) resins obtained by copolymerization of alpha-methylstyrene with other hydrocarbon comonomer such as for example styrene, vinyltoluene, and mixtures thereof;
- iii) phenolic-modified terpene resins such as for example resin product resulting from the condensation, in acidic medium of a terpene and a phenol;
- iv) Natural and modified rosin such as, for example, gun rosin, wood rosin, tall-oil rosin, distilled rosin, hydrogenated rosin, dimerized rosin and polymerized rosin;
- v) Glycerol and pentaerythritol esters of natural and modified rosin, such as, for example, the glycerol ester of pale wood rosin, the glycerol ester of hydrogenated rosin, the glycerol ester of polymerized rosin, the pentaerythritol ester of pale wood rosin, the pentaerythritol ester of hydrogenated rosin, the pentaerythritol ester of tall-oil rosin, and the phenolic modified pentaerythritol ester of rosin; and,
- vi) aliphatic and cycloaliphatic petroleum derived hydrocarbon resins, the latter resins resulting notably from the polymerization of monomers consisting primarily of aliphatic or cycloaliphatic olefins and diolefins. It also includes the hydrogenated aliphatic and cycloaliphatic petroleum hydrocarbon resins;
- vii) Aliphatic/aromatic petroleum derived hydrocarbon resins and the hydrogenated derivatives thereof;
- viii) mixtures thereof.

In particular, from those of types defined above, the following products may be mentioned:
- resin of type i) : polyterpene tackifiers from Arizone Chemical company sold under the trade name Sylvagum^{®} TR and Sylvares^{®} TR series (7115, 7125, M1115);
- resins of type ii) : Arkon^{®} series from Arakawa, and Hikotack^{®} series from Kolon which are produced from C9 monomers.
- resins of type iii) : Dertophene^{®} 1510 available from the company DRT : Dertophene^{®}H150, and Sylvarez^{®} TP95 available from Arizone Chemical which are phenolic-modified terpene resins;
- resins of type iv) : Foral^{®} DX from Pinova Inc., and Foral AX-E from Eastman company which are hydrogenated rosin resin.
- resins of type v) : Sylvalite^{®}RE 100 which is an ester or rosin and pentaerythritol available from Arizone Chemical;
- resins of type vi) : Escorez^{®}5400 which is a hydrogenated dicyclopentadiene resin available from Exxon Chemicals with a softening temperature of 100°C; Sukorez ^{®}SU210 sold by Kolon Company which is a partially hydrogenated aliphatic and cycloaliphatic petroleum derived hydrocarbon resin having a softening point of 110°C;
- resins of type vii) : Quintone^{®} DX390N sold by Zeon Company which is a non-hydrogenated aliphatic and aromatic petroleum derived hydrocarbon resin, having a softening point of 90C, and Sukorez^{®}SU400 sold by Kolon Company which is a hydrogenated aliphatic and aromatic petroleum derived hydrocarbon resin.

In a preferred embodiment, the hot melt adhesive composition does not comprise aromatic resins based on pure monomer streams of aromatic monomers such as for example vinyl toluene, styrene, alpha-methyl styrene, or indene. Examples of such resins are Kristalex^{®} or Plastolyn^{®} series sold by Eastman Chemical. According to this preferred embodiment, the hot melt adhesive exhibits advantageously adequate viscosity, adhesive and mechanical properties with low odor and reduced emission of VOC due to the absence of these particular tackifying resins.

### Stabilizers

The hot melt adhesive composition of the present invention may also include at least one stabilizer (antioxidant).

The hot melt adhesive composition comprises from about 0% to about 5 %, preferably from 0% to about 3 %, more preferably from about 0.1 % to about 3 %, more preferably from about 0.1% to 2 %, by weight of stabilizer(s) based on the total weight of the hot melt adhesive composition.

Suitable stabilizers are typically incorporated to help protect the polymers noted above, and thereby the total adhesive system from the effects of thermal and oxidative degradation which normally occurs during the manufacture and application of the adhesive, as well as in the ordinary exposure of the final product to the ambient environment.

Among the applicable stabilizers are for example high molecular weight hindered phenols and multifunction phenols, such as sulfur and phosphorous-containing phenols. Hindered phenols are well known to those skilled in the art and may be characterized as phenolic compounds that also contain sterically bulky radicals in close proximity to the phenolic hydroxyl group thereof. In particular, tertiary butyl groups generally are substituted onto the benzene ring in at least one of the ortho positions relative to the phenolic hydroxyl group. The presence of these sterically bulky substituted radicals in the vicinity of the hydroxyl group serves to retard its stretching frequency and correspondingly, its reactivity; this steric hindrance thus providing the phenolic compound with its stabilizing properties. Representative hindered phenols include: 1,3,5-trimethyl-2,4,6-tris(3-5-di-tert-butyl-4-hydroxybenzyl) benzene; pentaerythritol tetrakis-3(3,5-di-tert-butyl-4-hydroxyphenyl) propionate; n-octadecyl-3(3,5-di-tert-butyl-4-hydroxyphenyl) propionate, 4.4'-methylenebis(4-methyl-6-tert-butylphenol); 2,6-di-tert-butylphenol; 6-(4-hydroxyphenoxy)-2,4-bis(n-octylthio)-1,3,5-triazine; 2,3,6-tris(4-hydroxy-3,5-di-tert-butyl-phenoxy)-1,3,5-triazine; di-n-octadecyl-3,5-di-tert-butyl-4-hydroxybenzylphosphonate; 2-(n-octyl-thio)ethyl-3,5-di-tert-butyl-4-hydroxybenzoate and sorbitol hexa-3(3,5-di-tert-butyl-4-hydroxy-phenyl)propionate.

Suitable antioxidants are commercially available under the tradenames Irganox^{®} 1010 (tetrakis(methylene(3,5-di-ter-butyl-4-hydroxyhydrocinnamate)) methane), Irgafos^{®} 168 (tris(2,4-ditert-butylphenyl)phosphate) from BASF, and Chinox^{®} 1010 (pentaerythritol tetrakis(3-(3,5-di-tert-butyl-4-hydroxyphenyl) propionate)) from Shuangjian, Evernox^{®} 1010 and Everfos^{®} 168 from Everspring Chemical, Songnox^{®} 10 and 1680 from Songwon.

### Other additives

The hot melt adhesive composition may further comprise at least one additive, preferably in an amount of from 0% to 5 %, by total weight of the hot melt adhesive composition.

The additives may be selected from the group consisting of inert colorants (e.g. titanium dioxide), fillers (*e.g.* talc, calcium carbonate, clay, silica, mica, wollastonite, feldspar, aluminum silicate, alumina, hydrated alumina, glass microspheres, ceramic microspheres, thermoplastic microspheres and mixtures thereof), surfactants, additional polymers other than styrene block copolymers, crosslinking agents, nucleating agents, reactive compounds, fire-retardant mineral or organic agents, ultraviolet (UV) or infrared (IR) light absorbing agents, and UV or IR fluorescing agents. These optional auxiliary additives are well known in this art.

The additional polymers may be chosen from the group consisting of polyolefins, amorphous poly-alpha-olefins, ethyl-vinyl-acetate polymers (EVA), polyamides, and mixtures therefor. The hot melt adhesive may comprise from about 0% to about 5 % by weight of at least one additional polymer, by total weight of the composition.

### Hot melt adhesive composition

The viscosity is a Brookfield viscosity measured according to the standard method ASTM method D-3236, using a Brookfield viscometer of the type Spindle 27, at a temperature of about 177°C or 120°C.

The hot melt adhesive composition has a viscosity at 120°C higher than or equal to 30 000 mPa.s, preferably higher than or equal to 50 000 mPa.s, and even more preferably higher than or equal to 80 000 mPa.s.

The hot melt adhesive composition has a viscosity at 177°C lower than or equal to 5 000 mPa.s, preferably lower than or equal to 4 000 mPa.s, and even more preferably lower than or equal to 3 000 mPa.s.

The hot melt adhesive compositions according to the invention advantageously possess adequate viscosities which allow them to be applied at suitable temperature (such as for example at 160°C). Besides, the viscosity is advantageously not so low at 120°C which permits a better application and better homogeneity of the resulting adhesive structure. Indeed, at too low application temperature, if hot melt adhesives are too liquid, the fast cooling process lead to inhomogeneity of the adhesive structure.

The hot melt adhesive compositions according to the invention advantageously possess good processability (due to viscosity ranges at 177°C and 120°C) while exhibiting good adhesive and cohesion properties. Good adhesive properties is important to maintain contact between the absorbent material and substrate. Good internal cohesion ensures the adhesive will not break in particular in response to external forces during use. Thus high internal cohesion advantageously allows to maintain the absorbent core together at a dry state and during expansion while the core is wet.

These properties makes the hot melt adhesive compositions advantageously useful as core stabilization adhesive (for keeping the absorbent material of the core in place and maintain the integrity of the core structure especially when the absorbent article is in use).

### Process for preparing the hot melt adhesive composition

The invention also relates to a process for preparing the hot melt adhesive composition as described above.

The hot melt adhesive composition may be prepared by mixing the at least one styrenic block copolymer P, the at least one Fischer-Tropsch wax, in the molten state. Optional other ingredients or additives of the composition, as described above, may be mixed with the abovementioned components.

The hot melt adhesive composition of the present invention may be produced using any of the techniques known in the art. Preferably, the step of mixing is carried out at a temperature of from 130°C to 180°C. The ingredients are preferably mixed for at least several hours, typically at least 4 hours, and preferably from 4 to 6 hours.

The hot melt adhesive composition according to the invention can be prepared in presence of dioxygen (such as under air atmosphere), or preferably under inert atmosphere *e.g*. under carbon dioxide or nitrogen to limit potential degradation by oxidative reactions.

According to some embodiments, the process for preparing the hot melt adhesive composition according to the invention comprises:
- a first step of mixing the tackifying resin and the plasticizer(s), preferably with the antioxidant when present, at a temperature ranging from 150°C to 180°C, at least for a period of time long enough to melt all the tackifying resin,
- a second step of cooling the mixture to a temperature ranging from 130°C to 150°C,
- a third step of adding the styrenic block copolymer (A) into the mixture obtained in the previous step under stirring and heating at a temperature ranging from 130°C to 150°C, at least for a period of time long enough to melt all the styrenic block copolymer,
- a fourth step of adding the wax into the mixture obtained in the previous step under stirring and heating at a temperature ranging from 130°C to 150°C, at least for a period of time long enough to melt all the styrenic block copolymer.

Additionally, the process of the invention may comprise a step of applying vacuum to remove any entrapped air in the mixture, before or after any of the steps of process described previously.

Other useful optional ingredients or additives which may be present in the hot melt adhesive composition according to the invention, as described above, may be added at any step of the process according to the invention.

### Uses

The present invention relates to the use of the hot melt adhesive composition as defined herein, for the manufacture of disposable absorbent articles such as for example diapers, adult incontinent products, bed pads, sanitary napkins, medical dressings, bandages.

The present invention relates to the use of the hot melt adhesive composition as defined herein as core stabilization adhesive.

The present invention relates to a disposable absorbent article comprising the hot melt adhesive composition as defined above. The disposable absorbent article may be selected from the group consisting of diapers, adult incontinent products, bed pads, sanitary napkins, medical dressings, bandages, preferably a diaper.

The disposable absorbent article comprises an absorbent core which includes the core and optionally a core wrap.

The absorbent core may be manufactured in-line (i.e. in the disposable absorbent article) assembly line. Alternatively, the core may be manufactured off-line and then rolled-up or festooned for use at a later date or a different location.

The core can vary in design, shape. It can be shaped like a long rectangle or like a dog bone. It can be one continuous shape or can be broken into channels formed by for example the top sheet and the back sheet.

The absorbent core is generally centered within the article, and is firmly secured between the top sheet and the back sheet through an attachment means (e.g. adhesive).

The core can comprise different layer. The core typically includes absorbent materials capable of absorbing bodily fluids received from the top sheet. The core can include cellulose fibers (i.e. fluff), wood pulp, cotton, synthetic fibers, nonwoven, tissue, foam, superabsorbent polymers ("SAP") or any other absorbent material.

The hot met adhesive composition according to the invention may be applied as a coating (layer) or in the form of fibers.

The core wrap can be any material that wraps around the core to hold the components of the core in place. The core wrap generally wraps around the two longer sides of the core, leaving the ends open. The core wrap can be selected from the group consisting of nonwoven and tissue.

The hot melt adhesive composition can be used to hold the absorbent materials in place (in particular within the core wrap if present) and/or ensure integrity of the core structure in both the dry (unexpanded state) and wet (swollen state).

According to the present invention, by « comprised between x and y », or « ranging from x to y », it is meant a range wherein limits x and y are included. For example, the range "comprising between 1% and 3%" includes in particular 1% and 3%.

The following examples are given purely by way of illustration of the invention.

### EXPERIMENTAL PART

The following materials were used:
- Plasticizer 1: Nyflex^{®} 223 (naphtenic oil) from Nynas;
- Resin 1: Sukorez^{®} SU 525 (resin DCPD/C9 partially hydrogenated having a Ring and Ball softening point of 125°C) from Kolon;
- Resin 2: Sukorez^{®} SU 400 (resin DCPD/C9 partially hydrogenated having a Ring and Ball softening point of 100°C) from Kolon;
- Resin 3: Sukorez^{®} SU 420 (resin DCPD/C9 partially hydrogenated having a Ring and Ball softening point of 120°C) from Kolon;
- Polymer P1: VECTOR^{®} 4411 from TSRC is a linear SIS copolymer having 44% of styrene as styrene block and less than 1% of diblock content;
- Polymer P2 : TAIPOL^{®} 4202 from TSRC is a linear SBS copolymer having 40% of styrene as styrene block and less than 1% of diblock content;
- Wax 1 : SASOLWAX^{®} H1 from SASOL is a Fischer-Tropsch wax having a congealing point of 97°C and a needle penetration of 1 dmm;
- Antioxidant 1: Irganox^{®} 1010 (tetrakis(methylene(3,5-di-ter-butyl-4-hydroxyhydrocinnamate))methane) from BASF;
- Antioxidant 2: Irgafos^{®} 168 (tris(2,4-di-tert.-butylphenyl)phosphite) from BASF.

### A1 : preparation of the hot melt adhesives

The hot melt adhesive compositions in Table 1 below are prepared by mixing of its ingredients in the following order:
The polymer(s) and half of the resin(s) are added and mixed with the plasticizer(s) at 140°C until everything dissolved as a homogeneous mixture. Wax(es) and second half of the resin(s) are finally added at same temperature and mixed to get the final homogeneous adhesive mixture. At the end, the mixture is cooled down and collected to be used as it.

**Table 1 : hot melt adhesive compositions**

| **Raw material** | **HM1 (invention)** | **HM2 (invention)** | **HM3 (comparative)** | **HM4 (comparative)** | **HM5 (comparative)** |
|---|---|---|---|---|---|
| NYFLEX^{®} 223 | 20,0 | 20,0 | 20,0 | 20,0 | 20,0 |
| SUKOREZ^{®} SU400 | 10,0 | - | 10,0 | - | 10,0 |
| SUKOREZ^{®} SU420 | - | 11,5 | - | 11,5 | - |
| SUKOREZ^{®} SU525 | 34,5 | 35,0 | 34,5 | 35,0 | 29,5 |
| VECTOR^{®} 4411 | 32,5 | 14,25 | 34,5 | 16,25 | 29,5 |
| TAIPOL^{®} 4202 | - | 14,25 | - | 16,25 | - |
| SASOLWAX^{®} H1 | 2,0 | 4,0 | - | - | 10,0 |
| IRGANOX^{®} 1010 | 0,5 | 0,5 | 0,5 | 0,5 | 0,5 |
| IRGAFOS^{®} 168 | 0,5 | 0,5 | 0,5 | 0,5 | 0,5 |

### A2 : properties of the hot melt adhesive compositions

### Storage modulus (G') : Dynamical Mechanical Analysis (DMA)

A rheometer applied a shear stress to an adhesive and measured the resulting strain (shear deformation) response at constant temperature.

Equipment: DHR-1 Rheometer by TA Instrument, which applies a small oscillatory stress to achieve a constant oscillatory strain within the linear viscoelastic region of the adhesive (e.g. 1%).

General protocol: The adhesive was placed between a Peltier plate acting as lower, fixed plate and an upper plate with a radius R of 10 mm, which was connected to the drive shaft of a motor to generate the shear stress. The gap between both plates has a height H of 1,000 µm. The Peltier plate controls the temperature of the material (+/- 0.5°C).

Test execution:
- After GAP 0 at 80°C, setting the temperature to the start temperature (i.e. 150°C);
- placing the adhesive (1 cm3) on the Peltier plate;
- lowering the upper plate to a gap of 1,700 µm till its contacts the adhesive;
- removing excessive material and setting the temperature to 120°C;
- lowering the upper plate to a gap of 1,000 µm;
- setting the temperature to the start temperature of 120°C, the constant measurement strain to 1%, the constant oscillation frequency to 1 Hz and the cooling rate to 6°C/min (from 120°C to -10°C);
- after completing the test, melting the adhesive, lifting the upper plate and removing the adhesive from both the Peltier plate and the upper plate; and
- setting the temperature back to room temperature.

Result: Storage modulus were calculated all along the cooling step every 30 seconds. G' at 21°C and 60°C were determined accordingly.

### Brookfield viscosity

The Brookfield viscosity was measured according to the standard method ASTM D-3236, using a Brookfield viscometer of the type Spindle 27, at a temperature of 177°C .

### Results

| **properties** | **HM1 (invention)** | **HM2 (invention)** | **HM3 (comparative)** | **HM4 (comparative)** | **HM5 (comparative)** |
|---|---|---|---|---|---|
| Viscosity at 177°C (mPa.s) | 2279 | 2815 | 3150 | 3890 | 2150 |
| Viscosity at 120°C (mPa.s) | ≥ 30 000 mPa.s, | ≥ 30 000 mPa.s, | n.d | n.d | n.d |
| Storage modulus G' at 21°C (MPa) | 0.205 | 0.699 | 0.099 | 0.132 | N/A |
| Storage modulus G' at 60°C (MPa) | 0.087 | 0.122 | 0.068 | 0.077 | N/A |
| Compatibility | Ok | Ok | Ok | Ok | No |

| | | | | | |
|---|---|---|---|---|---|
| *N*/*A : Not Applicable since the hot melt is not homogeneous and cannot thus be analyzed through DMA analytical method as described above.* *n.d. : not determined* | | | | | |

The results of the table show that the two hot melt adhesive compositions HM1 and HM2 advantageously exhibit high storage modulus at 21°C (preferably higher than 0.150 MPa, even more higher than 0.2MPa), while keeping a viscosity at 177°C lower than 3 000 mPa.s which allows good processability during application of the hot melt. Thus, the hot melt adhesive composition HM1 and HM2 advantageously allow high internal cohesion and good processability.

The storage modulus obtained with comparative compositions HM3 (no wax), HM4 (no wax) and HM5 (10 wt% of wax) are lower than those obtained with the hot melt adhesive compositions of the invention HM1 and HM2.

Besides, the storage modulus of HM1 and HM2 at 60°C are also higher than that obtained with the comparative hot melt adhesive compositions HM3, HM4 and HM5.

## Claims

1. Hot melt adhesive composition comprising:
a) from 22% to 60% by weight of at least one styrene block copolymer (A) (designated as "SBC (A)") comprising:
• at least one linear styrene block copolymer (A1); and
• optionally at least one styrene diblock copolymer (A2);
said styrene block copolymer (A) having:
- a styrene content higher than or equal to 38% by weight based on the total weight of SBC (A); and
- a diblock content ranging from 0% to 10% by weight based on the total weight of SBC(A);
b) from 0.1% to 5% by weight of at least one wax having a congealing point higher than or equal to 60°C and a needle penetration at 25°C lower than or equal to 2 dmm;
wherein the hot melt adhesive composition has a viscosity at 120°C higher than or equal to 30 000 mPa.s, and a viscosity at 177°C lower than or equal to 5 000 mPa.s, wherein the congealing point, needle penetration and viscosity are determined by the methods defined in the description.

2. Hot melt adhesive composition according to claim 1, wherein the diblock content of the styrene block copolymer (A) ranges from 0% to 5 % by weight, more preferably from 0% to 2% by weight, and even more preferably from 0% to 1% by weight based on the total weight of SBC (A).

3. Hot melt adhesive composition according to anyone of claim 1 or 2, wherein the styrene block copolymer (A) has a styrene content ranging from 38% to 70% by weight, more preferably from 38% to 60% by weight, and even more preferably from 40% to 50% by weight based on the total weight of said styrene block copolymer (A).

4. Hot melt adhesive composition according to anyone of claims 1 to 3, wherein the linear styrene block copolymer (A1) is chosen from the following linear triblock copolymers: styrene-butadiene-styrene copolymer (SBS), styrene-isoprene-styrene copolymer (SIS), styrene-ethylene-butylene-styrene copolymer (SEBS), styrene-butadiene-butylene-styrene copolymer (SBBS), styrene-ethylene-propylene-styrene copolymer (SEPS), and any mixture thereof; the linear styrene block copolymer (A1) being more preferably selected from the group consisting of styrene-butadiene-styrene copolymer (SBS), styrene-isoprene-styrene copolymer (SIS), and mixtures thereof.

5. Hot melt adhesive composition according to anyone of claims 1 to 4, wherein the diblock styrene copolymer (A2) is chosen from the following diblock copolymers: styrene-butadiene diblock copolymer (SB), styrene-isoprene diblock copolymer (SI), styrene-ethylene-butylene diblock copolymer (SEB), styrene-ethylene-propylene diblock copolymer (SEP), styrene-butylene-butene (SBB), and mixtures thereof.

6. Hot melt adhesive composition according to anyone of claims 1 to 5, wherein the styrene block copolymer (A) has a melt flow index (200°C/5kg) higher than or equal to 20 g/10 min, preferably higher than or equal to 30 g/10 min.

7. Hot melt adhesive composition according to anyone of claims 1 to 6, wherein the total content of styrene block copolymer(s) (A) ranges from 25% to 50% by weight, more preferably from 25% to 40% by weight, and even more preferably from 27% to 37% by weight based on the total weight of the hot melt adhesive composition.

8. Hot melt adhesive composition according to anyone of claims 1 to 7, **characterized in that** it comprises from 1% to 5 % by weight of at least one wax as defined in claim 1, preferably from 1.5% to 4.0%, and even more preferably from 2.0% to 4.0% by weight based on the total weight of the hot melt adhesive composition.

9. Hot melt adhesive composition according to anyone of claims 1 to 8, wherein the wax is a synthetic wax, preferably a synthetic wax selected from the group consisting of Fischer-Tropsch waxes, polyolefin waxes, and mixtures thereof, the wax being more preferably a Fischer-Tropsch wax.

10. Hot melt adhesive composition according to anyone of claims 1 to 9, wherein the wax has a congealing point higher than or equal to 65°C, preferably higher than or equal to 70°C, and more preferably higher than or equal to 75°C.

11. Hot melt adhesive composition according to anyone of claims 1 to 10, wherein the needle penetration of the wax is lower than or equal to 1 dmm.

12. Hot melt adhesive composition according to anyone of claims 1 to 11, **characterized in that** it comprises from about 0% to about 40 %, preferably from about 5% to about 35 %, more preferably from about 10% to about 30 %, by weight of plasticizer(s) relative to the total weight of said hot melt adhesive composition.

13. Hot melt adhesive composition according to anyone of claims 1 to 12, wherein it further comprises at least one tackifying resin, preferably selected from the group consisting of:
- i) the homopolymers of terpene resins preferably having a softening point from 10°C to 120°C;
- ii) resins obtained by copolymerization of alpha-methylstyrene with other hydrocarbon comonomer such as for example styrene, vinyltoluene, and mixtures thereof;
- iii) phenolic-modified terpene resins;
- iv) Natural and modified rosin such as, for example, gun rosin, wood rosin, tall-oil rosin, distilled rosin, hydrogenated rosin, dimerized rosin and polymerized rosin;
- v) Glycerol and pentaerythritol esters of natural and modified rosin, such as, for example, the glycerol ester of pale wood rosin, the glycerol ester of hydrogenated rosin, the glycerol ester of polymerized rosin, the pentaerythritol ester of pale wood rosin, the pentaerythritol ester of hydrogenated rosin, the pentaerythritol ester of tall-oil rosin, and the phenolic modified pentaerythritol ester of rosin;
- vi) aliphatic and cycloaliphatic petroleum derived hydrocarbon resins, the latter resins resulting notably from the polymerization of monomers consisting primarily of aliphatic or cycloaliphatic olefins and diolefins;
- vii) Aliphatic/aromatic petroleum derived hydrocarbon resins and the hydrogenated derivatives thereof;
- viii) mixtures thereof.

14. Use of the hot melt adhesive composition according to anyone of claims 1 to 13, for the manufacture of disposable absorbent articles such as for example diapers, adult incontinent products, bed pads, sanitary napkins, medical dressings, bandages.

15. Use of the hot melt adhesive composition according to anyone of claims 1 to 13, as core stabilization adhesive.

16. Disposable absorbent article comprising the hot melt adhesive composition according to anyone of claims 1 to 13.

## Patentansprüche

1. Schmelzkleberzusammensetzung umfassend:
a) von 22 Gew.-% bis 60 Gew.-% an wenigstens einem Styrol-Blockcopolymer (A) (als "SBC (A)" bezeichnet) umfassend:
• wenigstens ein lineares Styrol-Blockcopolymer (A1); und
• gegebenenfalls wenigstens ein Styrol-Diblockcopolymer (A2);
wobei das Styrol-Blockcopolymer (A) aufweist:
- einen Styrolgehalt von höher als oder gleich 38 Gew.-%, bezogen auf das Gesamtgewicht von SBC (A); und
- einen Diblockgehalt in dem Bereich von 0 bis 10 Gew.-%, bezogen auf das Gesamtgewicht von SBC (A);
b) von 0,1 Gew.-% bis 5 Gew.-% an wenigstens einem Wachs mit einem Erstarrungspunkt von höher als oder gleich 60 °C und einer Nadelpenetration bei 25 °C von kleiner als oder gleich 2 dmm;
wobei die Schmelzkleberzusammensetzung eine Viskosität bei 120 °C von höher als oder gleich 30.000 mPa.s und eine Viskosität bei 177 °C von niedriger als oder gleich 5.000 mPa.s aufweist, wobei der Erstarrungspunkt, die Nadelpenetration und die Viskosität durch die in der Beschreibung definierten Verfahren bestimmt werden.

2. Schmelzkleberzusammensetzung nach Anspruch 1, wobei der Diblockgehalt des Styrol-Blockcopolymers (A) in dem Bereich von 0 Gew.-% bis 5 Gew.-%, bevorzugter von 0 Gew.-% bis 2 Gew.-% und noch bevorzugter von 0 Gew.-% bis 1 Gew.-%, bezogen auf das Gesamtgewicht von SBC (A), liegt.

3. Schmelzkleberzusammensetzung nach einem der Ansprüche 1 oder 2, wobei das Styrol-Blockcopolymer (A) einen Styrolgehalt in dem Bereich von 38 Gew.-% bis 70 Gew.-%, bevorzugter von 38 Gew.-% bis 60 Gew.-% und noch bevorzugter von 40 Gew.-% bis 50 Gew.-%, bezogen auf das Gesamtgewicht des Styrol-Blockcopolymers (A), aufweist.

4. Schmelzkleberzusammensetzung nach einem der Ansprüche 1 bis 3, wobei das lineare Styrol-Blockcopolymer (A1) ausgewählt ist aus den folgenden linearen Triblock-Copolymeren: Styrol-Butadien-Styrol-Copolymer (SBS), Styrol-Isopren-Styrol-Copolymer (SIS), Styrol-Ethylen-Butylen-Styrol-Copolymer (SEBS), Styrol-Butadien-Butylen-Styrol-Copolymer (SBBS), Styrol-Ethylen-Propylen-Styrol-Copolymer (SEPS) und einem beliebigen Gemisch davon; wobei das lineare Styrol-Blockcopolymer (A1) bevorzugter ausgewählt ist aus der Gruppe bestehend aus Styrol-Butadien-Styrol-Copolymer (SBS), Styrol-Isopren-Styrol-Copolymer (SIS) und Gemischen davon.

5. Schmelzkleberzusammensetzung nach einem der Ansprüche 1 bis 4, wobei das Diblock-Styrol-Copolymer (A2) ausgewählt ist aus den folgenden Diblock-Copolymeren: Styrol-Butadien-Diblock-Copolymer (SB), Styrol-Isopren-Diblock-Copolymer (SI), Styrol-Ethylen-Butylen-Diblock-Copolymer (SEB), Styrol-Ethylen-Propylen-Diblock-Copolymer (SEP), Styrol-Butylen-Buten (SBB) und Gemischen davon.

6. Schmelzkleberzusammensetzung nach einem der Ansprüche 1 bis 5, wobei das Styrol-Blockcopolymer (A) einen Schmelzflussindex (200°C/5kg) von höher als oder gleich 20 g/10 min, vorzugsweise höher als oder gleich 30 g/10 min, aufweist.

7. Schmelzkleberzusammensetzung nach einem der Ansprüche 1 bis 6, wobei der Gesamtgehalt an Styrol-Blockcopolymer(en) (A) in dem Bereich von 25 Gew.-% bis 50 Gew.-%, bevorzugter von 25 Gew.-% bis 40 Gew.-% und noch bevorzugter von 27 Gew.-% bis 37 Gew.-%, bezogen auf das Gesamtgewicht der Schmelzkleberzusammensetzung, liegt.

8. Schmelzkleberzusammensetzung nach einem der Ansprüche 1 bis 7, **dadurch gekennzeichnet, dass** sie von 1 Gew.-% bis 5 Gew.-% an wenigstens einem Wachs nach Anspruch 1, vorzugsweise von 1,5 Gew.-% bis 4,0 Gew.-% und noch bevorzugter 2,0 Gew.-% bis 4,0 Gew.-%, bezogen auf das Gesamtgewicht der Schmelzkleberzusammensetzung, umfasst.

9. Schmelzkleberzusammensetzung nach einem der Ansprüche 1 bis 8, wobei das Wachs ein synthetisches Wachs ist, vorzugsweise ein synthetisches Wachs ausgewählt aus der Gruppe bestehend aus Fischer-Tropsch-Wachsen, Polyolefinwachsen und Gemischen davon, wobei das Wachs bevorzugter ein Fischer-Tropsch-Wachs ist.

10. Schmelzkleberzusammensetzung nach einem der Ansprüche 1 bis 9, wobei das Wachs einen Erstarrungspunkt von höher als oder gleich 65 °C, vorzugsweise höher als oder gleich 70 °C und bevorzugter höher als oder gleich 75 °C aufweist.

11. Schmelzkleberzusammensetzung nach einem der Ansprüche 1 bis 10, wobei die Nadelpenetration des Wachses niedriger als oder gleich 1 dmm ist.

12. Schmelzkleberzusammensetzung nach einem der Ansprüche 1 bis 11, **dadurch gekennzeichnet, dass** sie von etwa 0 Gew.-% bis etwa 40 Gew.-%, vorzugsweise von etwa 5 Gew.-% bis etwa 35 Gew.-%, bevorzugter von etwa 10 Gew.-% bis etwa 30 Gew.-%, an Weichmacher(n), bezogen auf das Gesamtgewicht der Schmelzkleberzusammensetzung, umfasst.

13. Schmelzkleberzusammensetzung nach einem der Ansprüche 1 bis 12, ferner umfassend wenigstens ein klebrigmachendes Harz, vorzugsweise ausgewählt aus der Gruppe bestehend aus:
- i) den Homopolymeren von Terpenharzen, vorzugsweise mit einem Erweichungspunkt von 10 °C bis 120 °C;
- ii) Harzen, erhalten durch Copolymerisation von alpha-Methylstyrol mit anderen Kohlenwasserstoff-Comonomeren, wie z.B. Styrol, Vinyltoluol und Gemischen davon;
- iii) phenolisch modifizierten Terpenharzen;
- iv) natürlichem und modifiziertem Kolophonium, wie z.B. Waffenkophonium, Holzkolophonium, Tallölkolophonium, destilliertes Kolophonium, hydriertes Kolophonium, dimerisiertes Kolophonium und polymerisiertes Kolophonium;
- v) Glycerin- und Pentaerythritestern von natürlichem und modifiziertem Kolophonium, wie z.B. der Glycerinester von Harz von hellem Holz, der Glycerinester von hydriertem Kolophonium, der Glycerinester von polymerisiertem Kolophonium, der Pentaerythritester von Harz von hellem Holz, der Pentaerythritester von hydriertem Kolophonium, der Pentaerythritester von Tallölkolophonium und der phenolmodifizierte Pentaerythritester von Kolophonium;
- vi) aliphatischen und cycloaliphatische, Erdöl-abgeleiteten Kohlenwasserstoffharzen, wobei die letzteren Harze insbesondere aus der Polymerisation von Monomeren stammen, die hauptsächlich aus aliphatischen oder cycloaliphatischen Olefinen und Diolefinen bestehen;
- vii) aliphatischen/aromatischen Erdöl-abgeleiteten Kohlenwasserstoffharzen und den hydrierten Derivaten davon;
- viii) Gemischen davon.

14. Verwendung der Schmelzkleberzusammensetzung nach einem der Ansprüche 1 bis 13 zur Herstellung von absorptionsfähigen Einweggegenständen, wie z.B. Windeln, Inkontinenzprodukten für Erwachsene, Bettauflagen, Damenbinden, medizinischen Verbänden, Bandagen.

15. Verwendung der Schmelzkleberzusammensetzung nach einem der Ansprüche 1 bis 13 als Kernstabilisierungskleber.

16. Absorptionsfähiger Einweggegenstand umfassend die Schmelzkleberzusammensetzung nach einem der Ansprüche 1 bis 13.

## Revendications

1. Composition d'adhésif thermofusible comprenant :
a) de 22 % à 60 % en poids d'au moins un copolymère à blocs de styrène (A) (désigné par « SBC (A) ») comprenant :
• au moins un copolymère à blocs de styrène linéaire (A1) ; et
• éventuellement au moins un copolymère dibloc de styrène (A2) ;
ledit copolymère à blocs de styrène (A) ayant :
- une teneur en styrène supérieure ou égale à 38 % en poids sur base du poids total de SBC (A) ; et
- une teneur en dibloc allant de 0 à 10 % en poids sur base du poids total de SBC (A) ;
b) de 0,1 % à 5 % en poids d'au moins une cire possédant un point de congélation supérieur ou égal à 60 °C et une pénétration à l'aiguille à 25 °C inférieure ou égale à 2 dmm ;
la composition d'adhésif thermofusible possédant une viscosité à 120 °C supérieure ou égale à 30 000 mPa.s, et une viscosité à 177 °C inférieure ou égale à 5 000 mPa.s, le point de congélation, la pénétration à l'aiguille et la viscosité étant déterminés par les procédés définis dans la description.

2. Composition d'adhésif thermofusible selon la revendication 1, dans laquelle la teneur en dibloc du copolymère à blocs de styrène (A) est dans la plage de 0 % à 5 % en poids, plus préférablement de 0 % à 2 % en poids, et encore plus préférablement de 0 % à 1 % en poids par rapport au poids total de SBC (A).

3. Composition d'adhésif thermofusible selon l'une quelconque des revendications 1 ou 2, dans laquelle le copolymère à blocs de styrène (A) a une teneur en styrène allant de 38 % à 70 % en poids, plus préférablement de 38 % à 60 % en poids, et encore plus préférablement de 40 % à 50 % en poids par rapport au poids total dudit copolymère à blocs de styrène (A).

4. Composition d'adhésif thermofusible selon l'une quelconque des revendications 1 à 3, dans laquelle le copolymère à blocs de styrène linéaire (A1) est choisi parmi les copolymères triblocs linéaires suivants : copolymère styrène-butadiène-styrène (SBS), copolymère styrène-isoprène-styrène (SIS), copolymère styrène-éthylène-butylène-styrène (SEBS), copolymère styrène-butadiène-butylène-styrène (SBBS), copolymère styrène-éthylène-propylène-styrène (SEPS), et l'un quelconque de leurs mélanges ; le copolymère à blocs de styrène linéaire (A1) étant plus préférablement choisi dans le groupe constitué par un copolymère styrène-butadiène-styrène (SBS), un copolymère styrène-isoprène-styrène (SIS), et leurs mélanges.

5. Composition d'adhésif thermofusible selon l'une quelconque des revendications 1 à 4, dans laquelle le copolymère dibloc de styrène (A2) est choisi parmi les copolymères diblocs suivants : copolymère dibloc styrène-butadiène (SB), copolymère dibloc styrène-isoprène (SI), copolymère dibloc styrène-éthylène-butylène (SEB), copolymère dibloc styrène-éthylène-propylène (SEP), styrène-butylène-butène (SBB), et leurs mélanges.

6. Composition d'adhésif thermofusible selon l'une quelconque des revendications 1 à 5, dans laquelle le copolymère à blocs de styrène (A) a un indice de fluidité à chaud (200 °C/5 kg) supérieur ou égal à 20 g/10 min, de préférence supérieur ou égal à 30 g/10 min.

7. Composition d'adhésif thermofusible selon l'une quelconque des revendications 1 à 6, dans laquelle la teneur totale du ou des copolymère(s) à blocs de styrène (A) est dans la plage de 25 % à 50 % en poids, plus préférablement de 25 % à 40 % en poids et encore plus préférablement de 27 % à 37 % en poids par rapport au poids total de la composition d'adhésif thermofusible.

8. Composition d'adhésif thermofusible selon l'une quelconque des revendications 1 à 7, **caractérisée en ce qu'**elle comprend de 1 % à 5 % en poids d'au moins une cire telle que définie dans la revendication 1, de préférence de 1,5 % à 4,0 %, et encore plus préférablement de 2,0 % à 4,0 % en poids par rapport au poids total de la composition d'adhésif thermofusible.

9. Composition d'adhésif thermofusible selon l'une quelconque des revendications 1 à 8, dans laquelle la cire est une cire synthétique, de préférence une cire synthétique choisie dans le groupe constitué par les cires Fischer-Tropsch, les cires de polyoléfine et leurs mélanges, la cire étant plus préférablement une cire Fischer-Tropsch.

10. Composition d'adhésif thermofusible selon l'une quelconque des revendications 1 à 9, dans laquelle la cire a un point de congélation supérieur ou égal à 65 °C, de préférence supérieur ou égal à 70 °C, et plus préférablement supérieur ou égal à 75 °C.

11. Composition d'adhésif thermofusible selon l'une quelconque des revendications 1 à 10, dans laquelle la pénétration à l'aiguille de la cire est inférieure ou égale à 1 dmm.

12. Composition d'adhésif thermofusible selon l'une quelconque des revendications 1 à 11, **caractérisée en ce qu'**elle comprend d'environ 0 % à environ 40 %, de préférence d'environ 5 % à environ 35 %, plus préférablement d'environ 10 % à environ 30 %, en poids de plastifiant(s) par rapport au poids total de ladite composition d'adhésif thermofusible.

13. Composition d'adhésif thermofusible selon l'une quelconque des revendications 1 à 12, dans laquelle elle comprend en outre au moins une résine tackifiante, préférablement choisie dans le groupe constitué par :
- i) les homopolymères de résines terpéniques ayant, de préférence, un point de ramollissement de 10 °C à 120 °C ;
- ii) les résines obtenues par copolymérisation de l'alpha-méthylstyrène avec un autre comonomère hydrocarboné tel que par exemple le styrène, un vinyltoluène, et leurs mélanges ;
- iii) les résines de terpéniques modifiées de manière phénolique ;
- iv) une colophane naturelle et modifiée comme par exemple une colophane de pistolet, une colophane de bois, une colophane à base d'huile de queue, une colophane distillée, une colophane hydrogénée, une colophane dimérisée et une colophane polymérisée ;
- v) les esters de glycérol et de pentaérythritol de colophane naturelle et modifiée, tels que par exemple l'ester de glycérol de colophane de bois clair, l'ester de glycérol de colophane hydrogénée, l'ester de glycérol de colophane polymérisée, l'ester pentaérythritol de colophane de bois clair, l'ester pentaérythritol de colophane hydrogénée, l'ester pentaérythritol de colophane de queue, et l'ester de pentaérythritol de colophane modifiée de manière phénolique ;
- vi) les résines d'hydrocarbures dérivés du pétrole aliphatiques et cycloaliphatiques, les dernières résines résultant notamment de la polymérisation de monomères constitués majoritairement d'oléfines et dioléfines aliphatiques ou cycloaliphatiques ;
- vii) les résines d'hydrocarbures dérivés du pétrole aliphatiques/aromatiques et leurs dérivés hydrogénés ;
- viii) leurs mélanges.

14. Utilisation de la composition d'adhésif thermofusible selon l'une quelconque des revendications 1 à 13, pour la fabrication d'articles absorbants jetables tels que par exemple des couches, des produits pour l'incontinence pour adulte, des serviettes de lit, des serviettes hygiéniques, des pansements médicaux, des bandages.

15. Utilisation de la composition d'adhésif thermofusible selon l'une quelconque des revendications 1 à 13, en tant qu'adhésif de stabilisation de noyau.

16. Article absorbant jetable comprenant la composition d'adhésif thermofusible selon l'une quelconque des revendications 1 à 13.
